# EUROPEAN PATENT APPLICATION

(11) **EP 2 514 394 A1**
(43) Date of publication of application: **24.10.2012**
(21) Application number: 12164664.0
(22) Date of filing: 18.04.2012
(51) Int. Cl.: A61F 7/00

(54) **Temperature regulation system**

(30) Priority: 18.04.2011 IE 20110192
(71) Applicant: Eurolec Instrumentation Ltd., Co. Louth (IE)
(72) Inventor: Pointer, Ian Roger, Attleborough, Norfolk NR17 1SW (GB); Mears, William Thomas, Co. Louth (IE)
(74) Representative: Catesby, Olivia Joanne

(57) **Abstract**

A portable closed-loop system for temperature regulation of an area of the human body comprising:
a reservoir for a liquid,
an enveloping means for enveloping the area of the human body comprising at least one conduit in fluid communication with the reservoir; and
a control assembly configured to set the temperature of the liquid in the conduit;
wherein the conduit is arranged to uniformly regulate the temperature over the area enveloped by the enveloping means.

## Description

### Field of the Invention

The present invention relates to a temperature regulation system

### Background to the Invention

To improve patient prognosis following medical conditions such as heart attacks, strokes, head trauma, birth defects, etc there is a significant consensus amongst the medical profession that early intervention to cool the brain is greatly beneficial.

Induced hypothermia is commonly used to cool the patient's body temperature in order to achieve the improvements in prognosis. In addition, it is known that for patients undergoing treatments for cancers cooling the head may ease discomfort and assist in the retention of hair. However, present methods are generally invasive and involve the whole patient body. Cooling methods are generally restricted to a hospital environment, and involve whole body cooling with potential risks to other organs of the human body. In addition, many of the techniques used are body invasive techniques wherein liquid coolant is introduced through the nasal passages. Special coolants are also required as the coolants are introduced to the human body.

US 2002/0091431 A1 discloses a portable apparatus and method for cooling the brain of an infant in the event of prenatal hypoxic-ischemic injury. Headwear is fitted to the patient's head and a fluid is circulated though the headwear to cool the patient's brain. A reservoir containing the coolant is also supplied along with a warming means to maintain the temperature of the remainder of the patient's body. Brain temperature is assessed using a nasopharyngeal temperature probe or alternative means such as a temperature sensor in the auditory canal. The use of the cooling effect of evaporation arising from the coolant and gas required intake ports for both coolant and gas in combination with an exit port. To provide a continuous source of coolant and gas limits the portability of this system. In addition, the evaporation of coolant and gas results in the need to replace these features. By monitoring the patient's temperature the cooling effect is regulated, however such monitoring is inefficient.

It will be appreciated that following the cooling process it may be necessary to heat the cooled body part to return to a normal body temperature. Heating the human body is currently implemented using more traditional methods including foil wraps, hot water bottles blankets etc. As a result separate equipment is typically necessary to lower and then raise the body temperature.

### Object of the Invention

It is desirable to have a truly portable and compact closed loop system which improves the regulation of temperature as early as possible and which overcomes the disadvantages of the above systems.

It is therefore an object of the present invention to provide a self contained compact non-invasive and portable, simple to use electronically controlled system that can simply and accurately regulate the temperature of a specific area or body part to heat or cool the specific area or body part.

### Summary of the Invention

The invention described herein with reference to the appended claims discloses a portable closed-loop system for temperature regulation of an area of the human body comprising:
a reservoir for a liquid,
an enveloping means for enveloping the area of the human body comprising at least one conduit in fluid communication with the reservoir; and
a control assembly configured to set the temperature of the liquid in the conduit;
wherein the conduit is arranged to uniformly regulate the temperature over the area enveloped by the enveloping means.

The system is portable such that it can easily be transported to any location and can be used away from a hospital or other treatment area. It will be appreciated for example, that in the case of brain injury the earlier that cooling can be applied, the greater the benefits obtainable from cooling. Similarly, with soft tissue injuries, the portable nature of the present system allows for instant temperature regulation, i.e. cooling or heating of the injured area. The easily portable system facilitates continuity of cooling or heating from the earliest possible intervention. Furthermore, this system allows for continuity of intervention from the scene of any incident, through to a hospital environment and for as long as necessary thereafter.

The arrangement of the conduit within the enveloping means allows for a constant temperature over the area enveloped ensuring the that benefits of the cooling or heating is maximised.

The conduit may be arranged in a webbed configuration. The webbed configuration may comprise a plurality of concentric rings. Alternately, the webbed configuration may comprise a plurality of parallel paths. The webbed configuration may also comprise a snaked or spiral configuration.

The conduit may be defined by the enveloping means. It will be appreciated that the enveloping means may be moulded or otherwise formed such that channels are defined in the moulding process. Alternately, the conduit may be defined by a tube supported by the enveloping means in close proximity to the enveloped area. The tube may be supported in a channel defined in the enveloping means. In an alternative configuration the tubing may be mounted on the enveloping means.

The enveloping means may comprise a plurality of structural supporting ribs. It will be appreciated that the structural supporting ribs may allow the enveloping means maintain a desired shape, for example a shape corresponding to an area of the human body to be enveloped. The conduit may be mounted on or anchored to the structural ribs.

The portable closed loop system may further comprise flow and return tubing in fluid communication with the enveloping means and the reservoir. The flow and return tubing may be separate lengths of tubing or may be integral to a single length of tubing. The flow and return tubing may be insulated. It will be appreciated that the enveloping means may be detachable from the flow and return tubing. This allows alternative enveloping means to be used depending on the area of the human body.

The enveloping means may also comprise one or more quick fit connectors which allow additional components to be attached to the enveloping means. These additional attachments may include means for increasing the area to be cooled or heated. Additional attachments may also include lengths of tubing.

The enveloping means may comprise a head garment. Alternately, the enveloping means may be a bandage or strapping, support or brace. The enveloping means may comprise a thermometer or other temperature monitoring device for measuring the temperature of the area of the human body which is enveloped. In the embodiment described where the enveloping means comprises a head garment, the head garment may comprise a cannula feed. In the embodiment described where the enveloping means comprises a head garment quick fit connectors or the like may facilitate the attachment of additional accessories which extend the cooling or heating area to areas which can not be easily enveloped such as the nape of the neck. Such an accessory may also include a support or a brace, for example a carotid neck collar.

The portable closed loop system may further comprise means for securing the enveloping means to the body.

The portable closed loop system may further comprise an access point for accessing at least a part of the area of the body enveloped. It will be appreciated that an advantage of the access points is that treatment may be performed on the enveloped area while the enveloping means is in place.

The control assembly of the portable closed loop system may further comprise a pump for pumping the fluid between the reservoir and the enveloping means. The control assembly may further comprise a temperature controller. The control assembly may further comprise a temperature sensor for monitoring the temperature of the liquid in the conduit.

The portable closed loop system may further comprise means for determining the temperature of the enveloped area. Such a feature allows the enveloped area to be maintained at a closely regulated and desirable temperature.

The control assembly of the portable closed loop system in accordance with the invention may further comprise a heat exchange unit. The heat exchange unit may be configured to controllably alter the temperature of the liquid in the conduit.

The control unit may be further configured to use an internal or external power source.

A further embodiment of the present invention includes a body enveloping means for enveloping an area of the human body comprising:
at least one conduit for transporting a temperature controlled liquid in close proximity to the area of the human body;
a frame for supporting the at least one conduit in close proximity to the area of the human body; wherein the conduit is arranged such that the thermal transfer from the temperature controlled liquid to the area of the human body is uniform over the area enveloped by the body enveloping means.

The conduit may be arranged in a webbed configuration. The webbed configuration may comprise a plurality of concentric rings. The webbed configuration may comprises a plurality of parallel paths. The frame may comprise a plurality of structural supporting ribs.

The body enveloping means may further comprise a cover. The cover may further comprise a flexible material. The flexible material may also stretch to accommodate body parts of various diameters. The body enveloping means may be a head garment. The body enveloping means may also be a bandage, support, brace or the like. The head garment may comprises a cannula feed. The body enveloping means may comprise means for determining the temperature of the area of the human body enveloped. The means for determining may include a thermometer or other temperature sensor.

The body enveloping means may further comprise means for securing the body enveloping means to the area of the body. The body enveloping means may further comprise one or more connectors to which additional accessories such as a carotid neck collar.

A further embodiment of the present invention includes a control system for regulating a supply of liquid to a portable closed loop system as described above, comprising:
a heat exchange unit having a plurality of channels formed therein for transporting the supply of liquid through the heat exchange unit;
a temperature controller for setting a desired temperature of the heat exchange unit; and
means for monitoring the temperature of the heat exchange unit;
means for altering the setting of the temperature controller in response to a temperature reading from an area of the human body.

The control system may further comprise a heat sink. The control system may further comprise a fan. The control system may further comprise a thermoelectric heat pump. The heat pump may be attached to the heat exchange unit. The control system may further comprise insulation. The control system may further comprise a housing. It will be appreciated that the housing may encase the remaining elements of the control system. The housing may comprise an inlet and an outlet for allowing the liquid to pass through the control system. The housing may further comprise a handle. It will be appreciated that the handle can be used to assist in transporting the control unit. In use, the control unit may be hand held or mounted on a table top or other surface. The housing may further comprise a visual display unit. The visual display unit may be configured to display temperature or other statistics. The visual display unit may be configured to show the power status of the device, for example, whether the unit is powered internally by a battery or externally by a power source. The visual display unit may be used to display the remaining power in a battery or the voltage of the external voltage source. The housing may further comprise a connector for a cannula feed. The housing may also comprise a connector for connecting an external thermometer suitable for measuring the temperature of the area of the human body enveloped. The temperature reading from an area of the human body may be transmitted wirelessly to the control device. Alternatively the temperature reading may be obtained over a wired connection between a temperature sensor configured to measure the temperature of the area enveloped and the control device. The temperature reading may be transmitted from a temperature sensor mounted in the body enveloping means, on the human body or in close proximity to the area of the body enveloped by the body enveloping means.

### Brief Description of the Drawings

The present invention is described herein with reference by way of example only to the appended drawings, wherein
Fig. 1 is a perspective view of one embodiment of a control assembly in accordance with the present invention.
Fig.2 is view of the rear of the control assembly of Fig. 1
Fig. 3 is a perspective view of a heating or cooling device for use with the control assembly of Fig. 1 or 2.
Fig. 4 is a side view of the heating or cooling device of Fig. 3.
Fig. 5 is a detachable helmet device in accordance with one embodiment of the enveloping means of the system of the present invention.
Figure 6 is a view of the internal head-facing surface of the detachable helmet of Fig. 5.
Figure 7 is a diagrammatic representation of the liquid flow path among the components of a closed loop temperature regulation system in accordance with one embodiment of the present invention.

### Detailed Description of the Drawings

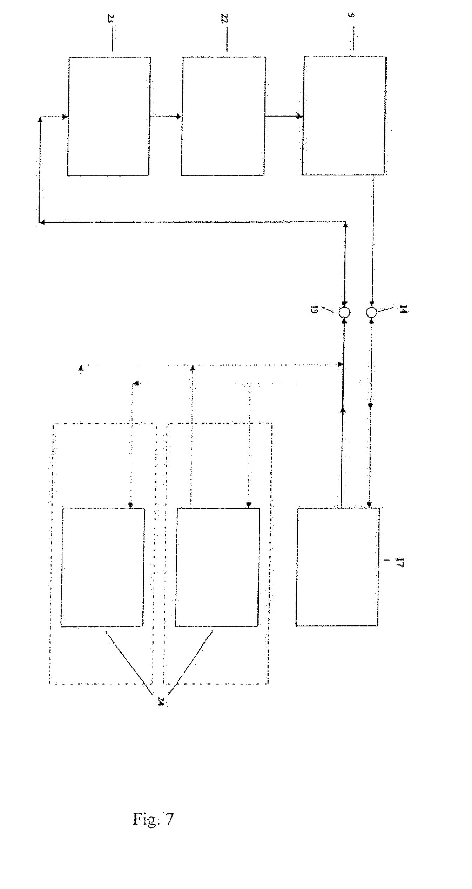

The present invention provides a closed loop system as depicted in Figure 7 for circulating fluid at accurately regulated temperatures through closed loop tubing held in close proximity to a body part, for example the human head, such that the temperature of that body part can be lowered or raised. The closed system includes a control assembly comprising a heat exchange unit 9, which is in communication with a pump 22 configured to pump a constant supply of liquid from a reservoir or storage unit, 23. The reservoir or storage unit may include a one-way air release valve (not shown) which can be used to release air locks from the system, and which can be automatically triggered to release air stored in the reservoir. The reservoir may be "topped up" or fed using connection valves, 13 or 14. These valves also serve as a connection point for a helmet 17, patch or bandage 24 which provides the carrier for tubing which conveys the liquid in close proximity to a part of the human body and thereby cools or heats the human body.

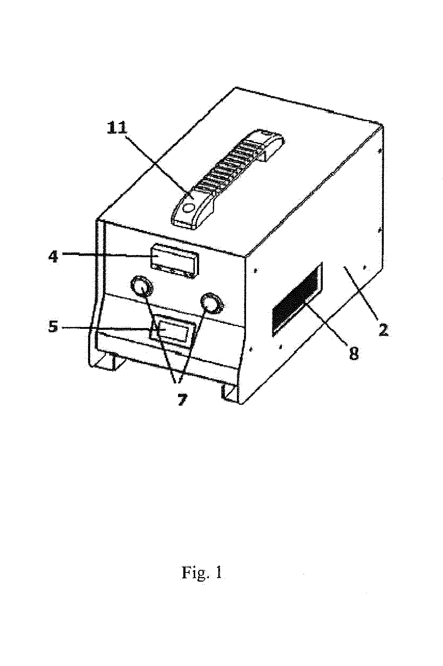

Individual components of the control assembly of the system of Figure 7 are shown in further detail in Figures 1 to 4. The control assembly can be used to regulate the temperature of the circulating fluid. For example the temperature may be raised or lowered using proportional, integral and derivative control to gradually increase of decrease the temperature by anticipating the set temperature and converging on this temperature without the possibility of overshooting. The control assembly alternatively may provide simple on-off control however on-off controllers have a wide hysteresis which could cause both overshooting and undershooting the desired temperature prior to settling at the desired temperature.

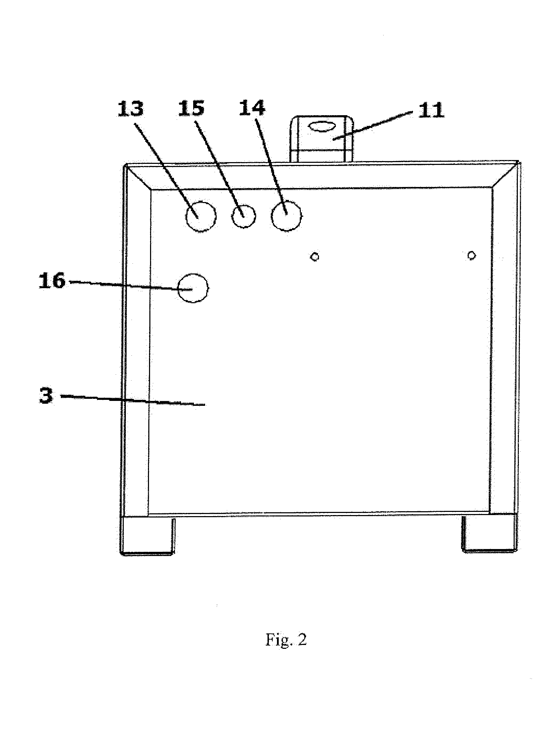

Figure 1 shows one embodiment of the control instrument which may form part of the closed loop system of the present invention. The control instrument of **Figure 1** includes an outer casing, 2, a temperature controller, 4, which may be a PID controller or the like, a patient thermometer, 5, switches, 7 for switching on and off the control unit and an external carrying handle 11. Connectors 13, 14 to and from control assembly are located on the rear of the assembly as shown in **Figure 2****.** In the embodiment shown in Figure 2, the rear panel, 3 of the control assembly includes an inlet connector, 13, an outlet connector, 14 a cannula feed, 15 and a connector 16 for an external power supply.

It will be appreciated that while an external power supply may be used, the control assembly may also be battery powered using rechargeable batteries or the like to increase portability. The control assembly may also be powered from an external 12V power source such as a vehicle battery or a suitable mains supply (110/240Vac). It will be appreciated that the drawings in Figures 1 and 2 are mere examples of the external casing of a suitable control device, and that alternative configurations are also envisaged.

In a preferred embodiment the temperature controller, 4 is fitted with a temperature sensor which is used to monitor the temperature of the liquid flowing through the controller. The temperature controller 4 can be a PID controller. The control assembly also includes a display which can be set to display temperature in °C or °F. In the configuration shown, the temperature display is included on the front of the control assembly. It will be appreciated that the thermometer may be remotely located and can transmit the temperature to the display.

In addition to monitoring the temperature of the liquid flowing through the control assembly, an external probe may be attached to the assembly which permits the monitoring of the patient's temperature, e.g. at the tympanic membrane. The desired patient temperature can be accurately achieved by monitoring the temperature of both the patient and the liquid and using these values in the controller 4 to give accurate and fast responses to the changing temperature of the patient and to ensure that undershooting or overshooting the desired temperature is minimised.

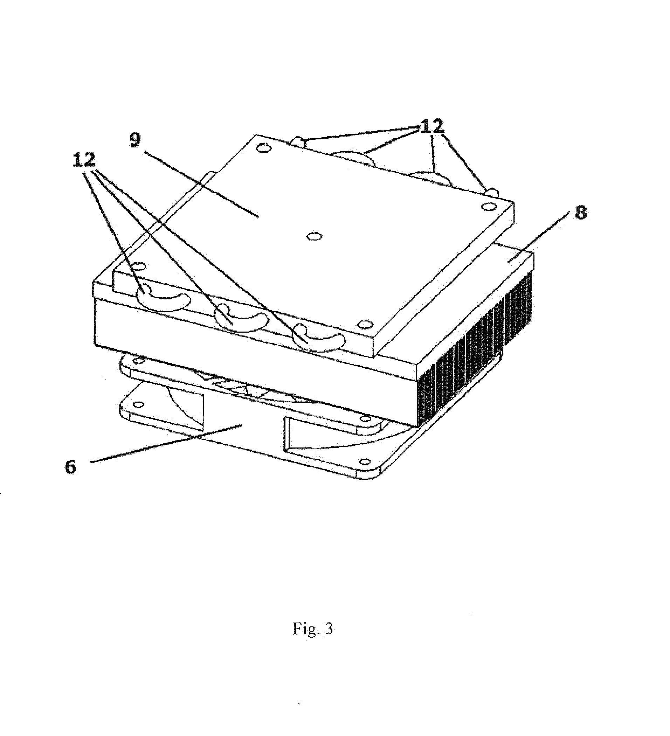

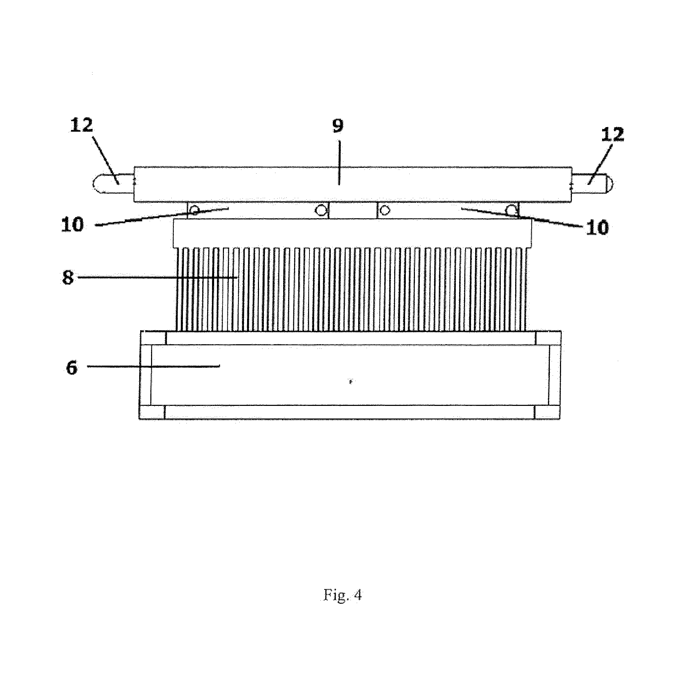

Internal to the control assembly as shown in Figures 1 and 2 in one embodiment of the present invention is a temperature control assembly as shown in **Figure 3** and **Figure 4****.**

The control assembly includes a fan, 6, a heatsink 8, a machined block or heat exchange unit 9 and interconnecting piping, 12 which may be copper piping or the like. One or more thermoelectric heat pumps 10 are included between the machined block 9 and the heatsink 8. The machined block is a heat exchanger to facilitate heating or cooling of circulating liquid. Also included is an electronic control circuit (not shown) that permits switching between internal and external power sources. The electronic control circuit is also configured to monitor the voltage level of any internal power supply and may also act as an interface between the temperature controller 4 and the thermoelectric heat pumps 10. The fan 6 enables cooling of control assembly and the heatsink to protect the components of the control assembly from overheating.

In the embodiment shown in Fig. 3, the precision-machined block, 9 includes a plurality of holes along the length of the block. These holes or cavities allow a fluid or liquid such as air or water to be circulated or pumped in and out through the machined block to ensure that it is sufficiently cooled or heated depending on the desired functionality. The temperature controller 4 communicates with a sensor (not shown) which is fitted into the machined block 9 for accurate temperature measurement.

The thermoelectric heat pumps 10 may be Peltier devices. Peltier devices are solid-state active heat pumps, which are configured to transfer heat from one side of the cooling device to the other. These devices may consist of two plates either side of thermocouples. Passing a current through this device warms one side and cools the alternate side. It will be appreciated that altering the polarity of the voltage applied to these devices will change the direction of the heat transfer.

The thermoelectric heat pumps, 10 are attached to the machined block 9. Thermal paste can be used to attach the heat pump and the machined block to ensure good thermal contact. The heatsink, 8 and fan 6 are used to dissipate the excess or unwanted heat from the heat pump, 10.

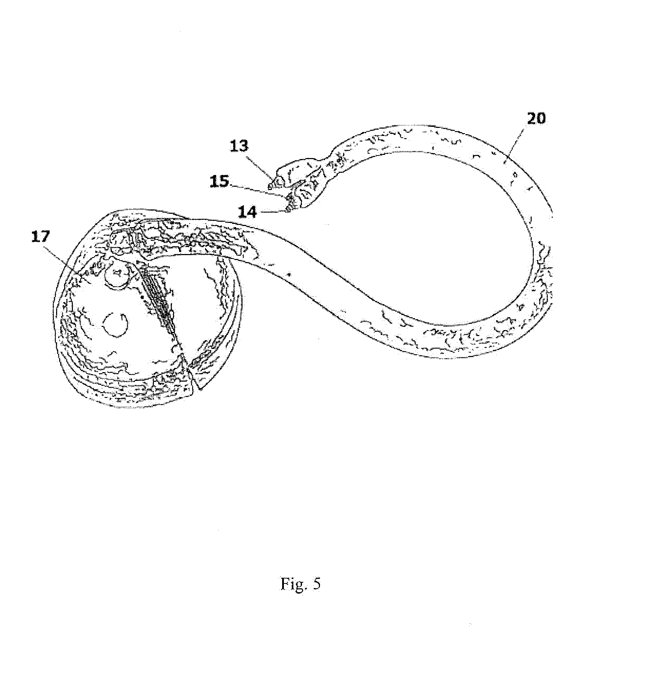

To minimize any unwanted heat transfer or loss and to efficiently maintain the desired temperature within the machined block, 9, insulation (not shown) is required. This insulation maintains a more constant and regulateable temperature of the liquid which is pumped through the pipes 12. The pipes 12 are connected via the outlet and inlet connector 13, 14 to the tubing or conduit which is held in close proximity to the part of the body which is to be heated or cooled by a specifically designed carrier. In one embodiment the conduit is held in a helmet or headpiece such as that shown in Figure 5. Alternatively, the conduit may be a bandage or support which can be applied to any part of the body.

Figure 5 shows an embodiment of a carrier in the form of a helmet which is detachable from the control assembly. The helmet can be placed on a patient's head to enable brain cooling. The helmet comprises an inlet connector 13 for connection to the corresponding connector on the controlling device and an outlet connector 14 for connection to the corresponding connector 14 on the controlling device. The connectors may include special fitting with a seal or quick fit connector which locks in place and releases an internal non-return valve. Removing the connectors automatically seals the non-return valve, thus ensuring a negligible loss of coolant in the closed system.

The helmet of Figure 5 may also comprise a cannula feed, 15 for connection to the corresponding cannula feed on the controlling device. The helmet 17 may be made of neoprene or similar flexible material. Extending between the helmet and the connectors is a section of flow and return tubing 20. In an embodiment of the present invention, the flow and return tubing is insulated. The insulation may comprise a flexible stretchable material which may be attached to and detached from the helmet.

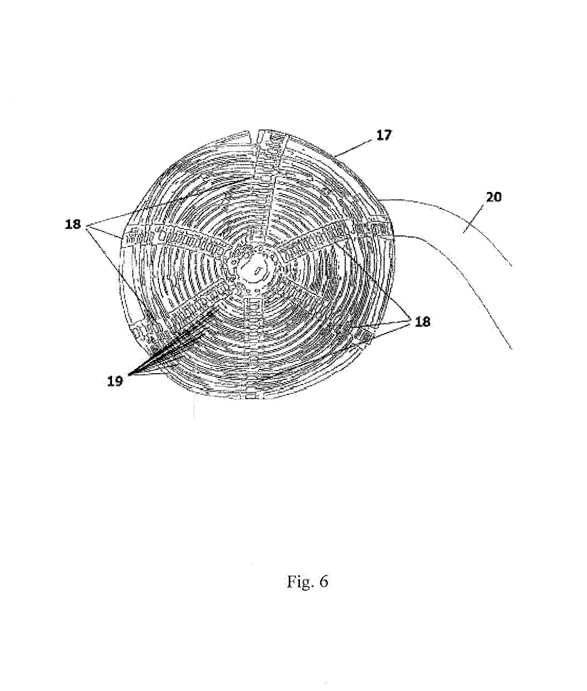

The internal structure of the helmet 17 is shown in **Fig. 6** and comprises a concentric pattern of tubing 19 that circulates the temperature-controlled liquid over the patient's head. The concentric pattern is a flexible spiderweb structure which carries the tubing to deliver the coolant as closely as possible to the different head contours to optimise the thermal transfer effect to the skull or elsewhere on the body if required. A spine moulding structure, frame or supporting rib structure 18 is provided to maintain the shape of the helmet and provide structure to the helmet. The spine moulding structure also functions to support the tubing in the desired concentric pattern. The helmet can be detached from the flow and return tubing 20. As shown in Fig 6, the tubing is flexible tubing that may be silicon tubing or the like.

In such closed loop system of Figure 7 the device maintains its own reservoir 23 to continuously supply the temperature controlled liquid, which may be a specific coolant via the tubing to the required part of the body. The liquid is continuously reused, thereby increasing the efficiency. The tubing is attached to the inside of the external helmet. It will be appreciated that the tubing may be integral to the helmet, however this integration would significantly reduce the flexibility in terms of interchangeability and the ability of the temperature controlled liquid to ensure good thermal conductivity with the different contours of various patients heads.

The tubing can be secured to the underside of the helmet using the spine structure to create concentric circles of tubing and ensure good thermal contact on the head and nape of the neck. To secure the helmet in place on the patients head Velcro™ may be used to hold the helmet in place. Alternative strapping structures may be used to secure the helmet to the patients chin to optimise thermal contact.

In a preferred embodiment the helmet or headpieces is configured such that it can be rotated through 360 degrees, to permit suitable alignment of the helmet. For example such alignment may allow access points to the surface of the head. Additional access points can be provided in the external surface of the helmet to allow access to the surface of the patient's head without needing to remove the helmet. It will be appreciated that the ability to rotate the helmet allows the access points to be moved around the patient's head without removing the helmet.

In one embodiment of the present invention the helmet is fed with lengths of flow and return tubing. The tubes are connected to the control instrument, 3 using inlet and outlet couplings. Shut off or non return valves may also be included on both of these connections to prevent any leakage. In addition, the versatile design utilising connectors with non-return valves will permit the simple interchange of different size helmets or head pieces or other carriers for the tubes. It will be appreciated that additional attachments for the helmets or head pieces may also include nape of the neck and collar options directed to brain stem and carrotted artery cooling) which extends the cooling/heating surfaces. The carriers for the tubing may also take the form of a heating or cooling pad for treatment of sporting or similar injuries.

It will be appreciated that while shown in figures 5 and 6 as having a circular cross section, the tubing can have any cross section, including oval or rectangular.

An optional cannula feed, 15 such as that shown in Fig. 5 may be used to permit a supply of chilled air from the control device to be directed to the nasal passages to enhance the cooling process.

The embodiment of carrier described above is specifically directed to the covering of the head, however, it will be appreciated that the invention extends to carriers of alternative forms designed and suitable for application to alternate areas of the body, for example, the knee or elbow joints.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

## Claims

1. A portable closed-loop system for temperature regulation of an area of the human body comprising:
a reservoir (23) for a liquid,
an enveloping means (24, 17) for enveloping the area of the human body comprising at least one conduit in fluid communication with the reservoir; and
a control assembly configured to set the temperature of the liquid in the conduit;
wherein the conduit is arranged to uniformly regulate the temperature over the area enveloped by the enveloping means.

2. The portable closed loop system according to Claim 1 wherein the conduit is arranged in a webbed configuration.

3. The portable closed loop system according to Claim 2 wherein the webbed configuration comprises a plurality of concentric rings.

4. The portable closed loop system according to claim 2 wherein the webbed configuration comprises a plurality of parallel paths.

5. The portable closed loop system according to any of Claims 1 to 3 wherein the conduit is defined within the enveloping means (17, 24).

6. The portable closed loop system according to any of Claims 1 to 4 wherein the conduit is defined within a tube supported by the enveloping means (17,24) in close proximity to the enveloped area.

7. The portable closed loop system according to any previous claim wherein the enveloping means comprises a plurality of structural supporting ribs (18).

8. The portable closed loop system according to any previous claim further comprising flow and return tubing (20) in fluid communication with the enveloping means (17,24) and the reservoir (23).

9. The portable closed loop system according to Claim 8 wherein the flow and return tubing (20) is insulated.

10. The portable closed loop system according to Claim 8 or 9 wherein the enveloping means (17, 24) is detachable from the flow and return tubing (20).

11. The portable closed loop system according to any previous claim wherein the enveloping means comprises a head garment (17).

12. The portable closed loop system according to any previous claim further comprising means for securing the enveloping means to the body.

13. The portable closed loop system according to any previous claim further comprising means for determining the temperature of the enveloped area.

14. A body enveloping means (17, 24) for enveloping an area of the human body comprising:
at least one conduit for transporting a temperature controlled liquid in close proximity to the area of the human body;
a frame for supporting the at least one conduit in close proximity to the area of the human body; wherein the conduit is arranged such that the thermal transfer from the temperature controlled liquid to the area of the human body is uniform over the area enveloped by the body enveloping means.

15. A control system for regulating a supply of liquid to a portable closed loop system according to any of Claims 1 to 13 comprising:
a heat exchange unit (9) having a plurality of channels formed therein for transporting the supply of liquid through the heat exchange unit;
a temperature controller (4) for setting a desired temperature of the heat exchange unit; and
means for monitoring the temperature of the heat exchange unit (9);
means for altering the setting of the temperature controller in response to a temperature reading from an area of the human body.
